# EUROPEAN PATENT APPLICATION

(11) **EP 4 730 400 A1**
(43) Date of publication of application: **22.04.2026**
(21) Application number: 24823352.0
(22) Date of filing: 10.06.2024
(51) Int. Cl.: H01L 31/12

(54) **REFLECTIVE OPTICAL SENSOR**

(30) Priority: 15.06.2023 JP 2023098321
(71) Applicant: Dexerials Corporation, Shimotsuke-shi, Tochigi 323-0194 (JP)
(72) Inventor: TATEKOUJI, Akihiro, Shimotsuke-shi, Tochigi 323-0194 (JP); YANAGASE, Masashi, Shimotsuke-shi, Tochigi 323-0194 (JP)
(74) Representative: Ter Meer Steinmeister & Partner
(86) International application number: PCT/JP2024/021093
(87) International publication number: WO 2024/257736

(57) **Abstract**

A reflective optical sensor includes a substrate; a light-emitting element and a light-receiving element both of which are provided on the substrate; and a light-shielding wall provided between the light-emitting element and the light-receiving element. A distance dₑ between the light-emitting element and the light-shielding wall, a distance dᵣ between the light-receiving element and the light-shielding wall, and a thickness t of the light-shielding wall satisfy the following relationship: dₑ + dᵣ < t. A shortest distance between a region that does not belong to any of a region, in which light emitted from the light-emitting element does not reach a measurement target, and a region, in which light reflected or scattered by the measurement target does not enter the light-receiving element, and a sensor surface in which the reflective optical sensor and the measurement target are in contact is 0.2 mm or greater and 0.3 mm or less.

## Description

### TECHNICAL FIELD

The present invention relates to a reflective optical sensor.

### BACKGROUND ART

As an apparatus for optically and non-invasively detecting biological information or the like using a reflective optical sensor, for example, a pulse oximeter for measuring blood oxygen saturation and a blood glucose meter for measuring blood glucose concentration, are known. A reflective optical sensor detects a change in an amount of received light as biological information by a light-emitting element, such as a light-emitting diode (LED), irradiating a fingertip or the like with light and a light-receiving element, such as a photodiode receiving reflected light from a living body (specifically, blood in a blood vessel), as a measurement target.

As a photoreflector having two light-emitting elements and one light-receiving element in one package, Patent Document 1 discloses a photosensor including a photoreflector having, as illustrated in FIG. 8, light-emitting elements (12) and a light-receiving element (13) both mounted on a substrate (11), a translucent resin (15) for sealing these elements, and a light-shielding resin (2), wherein the light-shielding resin (2) forms a light-shielding wall (2a) between the light-emitting element (12) and the light-receiving element (13), and eaves (2b) connected to the light-shielding wall (2a) are formed to narrow the light-emitting surface above the light-emitting elements (12).

### RELATED ART DOCUMENTS

### PATENT DOCUMENT

Patent Document 1: International Publication No. WO 2017/094089

### SUMMARY OF THE INVENTION

### PROBLEMS TO BE SOLVED BY THE INVENTION

However, although the conventional photoreflector disclosed in Patent Document 1 is designed to efficiently irradiate blood flowing through a blood vessel in a measurement target organism by using the eaves to limit the emission angle of light from the light-emitting elements, it does not restrict the incident angle of light entering the light-receiving element. As a result, signals reflected by the surface of the organism are also detected, generating noise in measurement signals.

The present invention is provided to solve the conventional problems and to achieve the following object of the present invention: to provide a reflective optical sensor having a simple structure and in which noise due to light from the surface of a measurement target is reduced.

### MEANS FOR SOLVING THE PROBLEMS

Means for solving the problems are as follows.
<1> A reflective optical sensor includes: a substrate; a light-emitting element and a light-receiving element, both of which are provided on the substrate; and a light-shielding wall provided between the light-emitting element and the light-receiving element. A distance (dₑ) between the light-emitting element and the light-shielding wall and a distance (dᵣ) between the light-receiving element and the light-shielding wall, and a thickness (t) of the light-shielding wall satisfies the following relationship dₑ + dᵣ < t. A shortest distance between a region that does not belong to any of a region in which light emitted from the light-emitting element does not reach a measurement target and a region in which light reflected or scattered by the measurement target does not enter the light-receiving element, and a sensor surface in which the reflective optical sensor and the measurement target are in contact, is 0.2 mm or greater and 0.3 mm or less.
<2> In the reflective optical sensor according to <1>, the light-emitting element is two or more light-emitting elements provided next to each other, and emission wavelengths of the two or more light-emitting elements are different from each other.
<3> The reflective optical sensor according to <1> or <2> further includes a light-shielding frame that, together with the light-shielding wall, separates the light-emitting element and the light-receiving element, the light-shielding frame being provided on the substrate. A shape of each section defined by the light-shielding wall and the light-shielding frame is a chamfered square columnar shape.
<4> In the reflective optical sensor according to claim 3, a thickness of the light-shielding frame is smaller than a thickness of the light-shielding wall.
<5> In the reflective optical sensor according to any one of <1> through <4>, the substrate is a glass epoxy substrate having a light transmittance of 1% or less through a thickness of the substrate. The light-shielding wall is a glass epoxy substrate having a light transmittance of 1% or less with through a thickness of the light-shielding wall. The light-shielding wall is provided on the substrate via a light-shielding adhesive.

### EFFECTS OF THE INVENTION

According to the present invention, it is possible to solve the above-described conventional problems, achieve the above-described object, and provide a reflective optical sensor having a simple structure and in which noise due to light from a surface of a measurement target is reduced.

### BRIEF DESCRIPTION OF THE DRAWINGS

[FIG. 1] FIG. 1 is a schematic cross-sectional view illustrating an example of a reflective optical sensor according to the present embodiment.
[FIG. 2] FIG. 2 is a schematic top view illustrating an example of the reflective optical sensor according to the present embodiment.
[FIG. 3] FIG. 3 is a schematic cross-sectional view illustrating a non-sensing distance of the reflective optical sensor according to the present embodiment.
[FIG. 4] FIG. 4 is a schematic cross-sectional view illustrating a non-sensing distance of a reflective optical sensor disclosed in prior art.
[FIG. 5] FIG. 5 is a schematic top view illustrating another example of the reflective optical sensor according to the present embodiment.
[FIG. 6] FIG. 6 is a schematic top view illustrating yet another example of the reflective optical sensor according to the present embodiment.
[FIG. 7] FIG. 7 is a schematic top view illustrating yet another example of the reflective optical sensor according to the present embodiment.

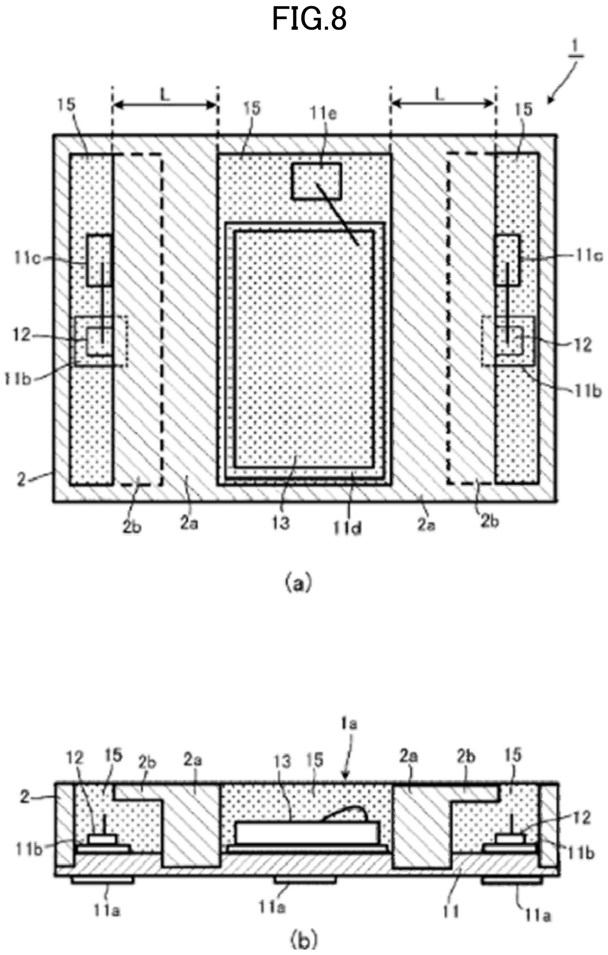

[FIG. 8] FIG. 8 is a schematic view illustrating an example of a reflective optical sensor disclosed in prior art.
[FIG. 9] FIG. 9 is a schematic view illustrating an example of a reflective optical sensor disclosed in other prior art.

### DESCRIPTION OF EMBODIMENTS

### (Reflective Optical Sensor)

A reflective optical sensor of the present invention includes a substrate; a light-emitting element and a light-receiving element, both of which are provided on the substrate; and a light-shielding wall provided between the light-emitting element and the light-receiving element. A distance dₑ between the light-emitting element and the light-shielding wall, a distance dᵣ between the light-receiving element and the light-shielding wall, and a thickness t of the light-shielding wall satisfy the following relationship: dₑ + dᵣ < t. A shortest distance, between a region that does not belong to any of a region in which light emitted from the light-emitting element does not reach a measurement target and a region in which light reflected or scattered by the measurement target does not enter the light-receiving element, and a sensor surface in which the reflective optical sensor and the measurement target are in contact, is 0.2 mm or greater and 0.3 mm or less.

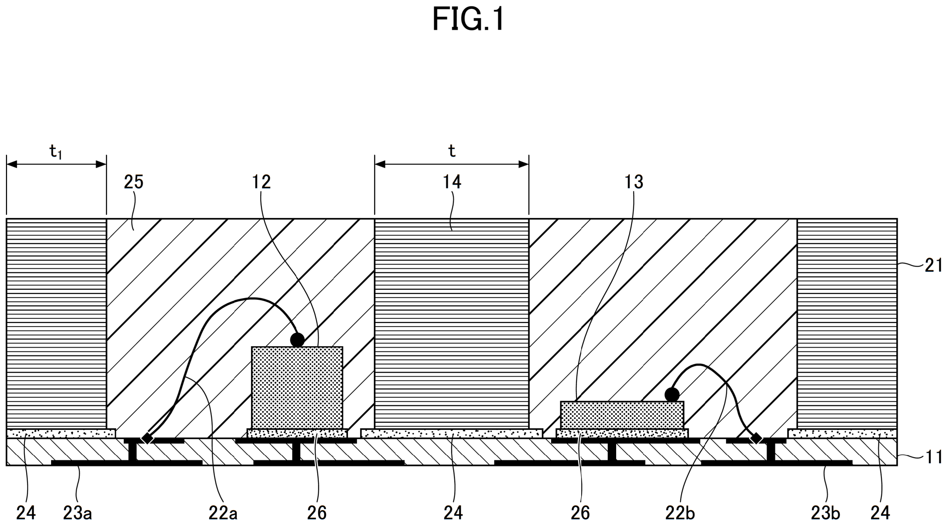

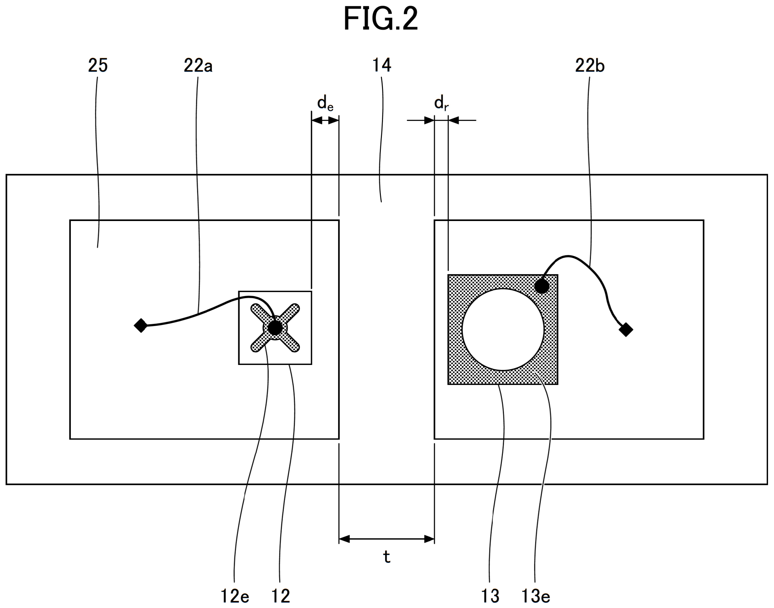

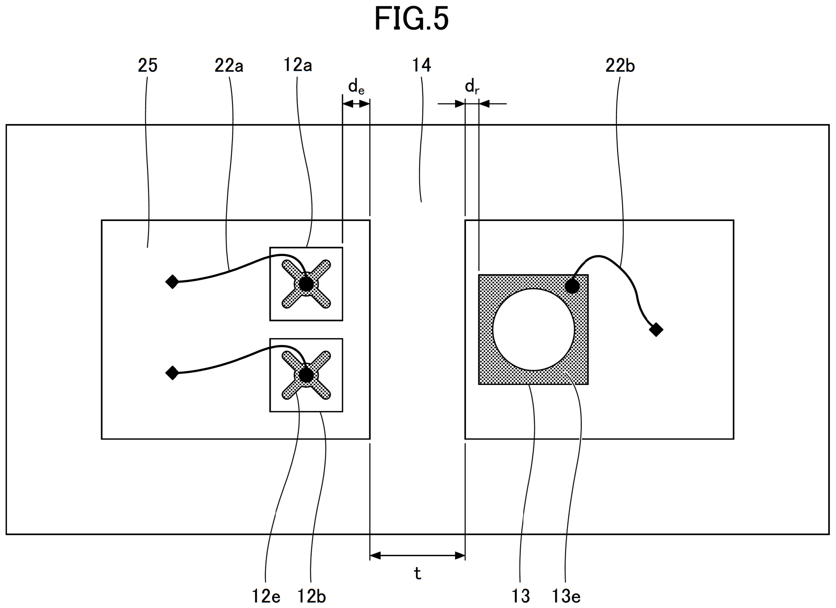

An example of the reflective optical sensor of the present embodiment will be described with reference to the drawings. FIG. 1 is a schematic cross-sectional view illustrating an example of a reflective optical sensor of the present embodiment, and FIG. 2 is a schematic top view of the reflective optical sensor of the present embodiment. As illustrated in FIGS. 1 and 2, the reflective optical sensor 10 includes a substrate 11, a light-emitting element 12 and a light-receiving element 13 that are both provided on the substrate 11, and a light-shielding wall 14 provided between the light-emitting element 12 and the light-receiving element 13. In the present embodiment, one light-emitting element 12 is provided, but two or more light-emitting elements may be provided as illustrated in FIG. 5, etc., which will be described later.

The reflective optical sensor 10 further includes a conductive wire 22a and a conductive portion 23a for supplying electric power to an electrode 12e of the light-emitting element 12 from the outside, and a conductive wire 22b and a conductive portion 23b for outputting photocurrent from an electrode 13e of the light-receiving element 13 to the outside. The light-emitting element 12 and the light-receiving element 13 are electrically connected to the conductive wire 22 and the conductive portion 23, thereby being enabled to supply electric power and output photocurrent. While the light-emitting element 12 and the light-receiving element 13 are provided next to the light-shielding wall 14, the conductive portions 23a and 23b to which the conductive wires 22a and 22b are connected are disposed and connected at positions away from the light-shielding wall 14.

The reflective optical sensor 10 further includes a light-shielding frame 21 provided on the substrate 11 to separate, together with the light-shielding wall 14, the light-emitting element 12 and the light-receiving element 13. The light-shielding wall 14 and the light-shielding frame 21 are provided on the substrate 11 via an adhesive layer 24 having light-shielding properties. The section for housing the light-emitting element 12 and the section for housing the light-receiving element 13 are filled with a transparent resin 25. The transparent resin 25 allows transmission of light radiated by the light-emitting element 12 and light reflected or scattered by a measurement target, and is an epoxy resin, for example. The transparent resin 25 seals the light-emitting element 12 and the light-receiving element 13, for example, to protect them from shock, moisture, or other undesirable factors. The surface (upper surface) of the transparent resin 25 is formed flat at the same height as the light-shielding wall 14 and the light-shielding frame 21, and forms a sensor surface that is brought into contact with a measurement target. The light-emitting element 12 and the light-receiving element 13 are provided on the substrate 11 via an optional adhesive layer 26.

As illustrated in FIGS. 1 and 2, in the reflective optical sensor 10, the distance dₑ between the light-emitting element 12 and the light-shielding wall 14, the distance dᵣ between the light-receiving element 13 and the light-shielding wall 14, and the thickness t of the light-shielding wall 14 satisfy the following relationship: dₑ + dᵣ < t. In other words, the light-emitting element 12 and the light-receiving element 13 are next to each other via the light-shielding wall 14 so as to satisfy dₑ + dᵣ < t. This means that the light-emitting element 12 and the light-receiving element 13 are disposed close to the light-shielding wall 14, and the light-shielding wall 14 having a thickness t that is relatively thick with respect to the distance dₑ and the distance dᵣ is provided between the light-emitting element 12 and the light-receiving element 13.

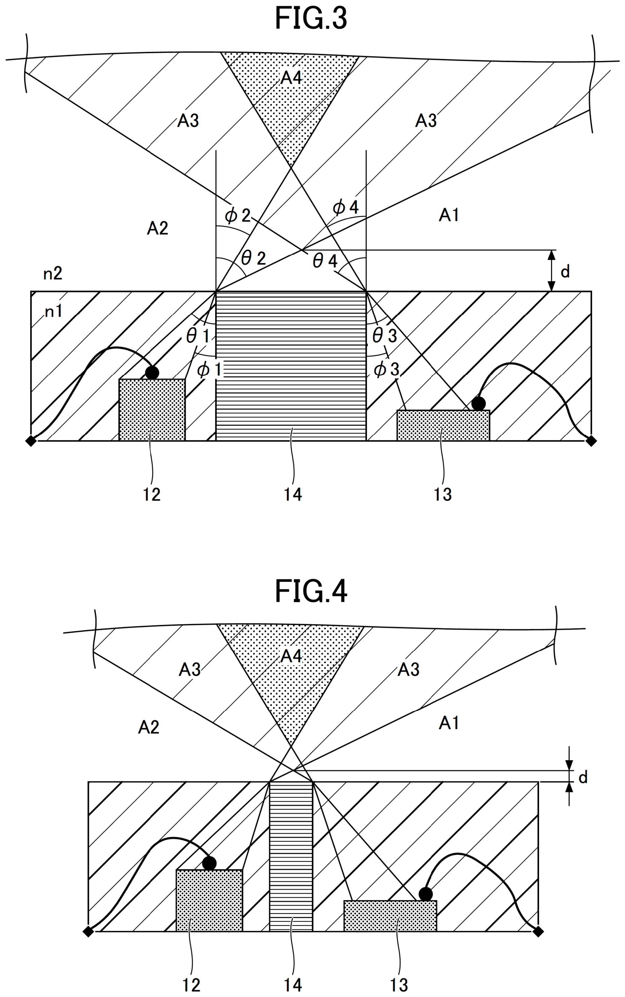

FIG. 3 is a schematic cross-sectional view illustrating a non-sensing distance of the reflective optical sensor of the present embodiment, and FIG. 4 is a schematic cross-sectional view illustrating a non-sensing distance of a reflective optical sensor disclosed in prior art. As illustrated in FIG. 3, a region A1 in which light emitted from the light-emitting element 12 does not reach a measurement target and a region A2 in which light reflected or scattered by a measurement target does not enter the light-receiving element 13 can be regarded as regions that the reflective optical sensor does not sense (non-sensing regions). A shortest distance d (also referred to as a "non-sensing distance") between a region (hereinafter referred to as the "region A3") that does not belong to any of the non-sensing regions A1 and A2 and a sensor surface where the reflective optical sensor 10 and the measurement target contact each other is equal to or greater than 0.2 mm and equal to or less than 0.3 mm. Herein, the shortest distance d between the region A3 and the sensor surface is a length (shortest distance) between the portions where the region A3 and the sensor surface are closest to each other, and is defined as a distance d between an intersection point where the boundary line of the region A1, which forms an angle θ2 with the side surface of the light-shielding wall 14, intersects with the boundary line of the region A2, which forms an angle θ4 with the side surface of the light-shielding wall 14, and the sensor surface of the reflective optical sensor in FIG. 3.

As illustrated in FIG. 3, when the reflective optical sensor 10 satisfies the relationship dₑ + dᵣ < t, the size of the area where the non-sensing region A1 and the non-sensing region A2 overlap each other can be increased as compared with the conventional reflective optical sensor disclosed in the prior art and illustrated in FIG. 4 that does not satisfy the relationship dₑ + dᵣ < t, and therefore, the non-sensing distance d can be set in a range of 0.2 mm or greater and 0.3 mm or less.

Herein, for example, in the case of detecting biological information in blood, such as blood oxygen saturation or blood glucose concentration with the reflective optical sensor 10, the sensor surface of the reflective optical sensor 10 and a living body serving as a measurement target contact each other. The structure of the skin of a living body is divided from the surface into three layers, which are the epidermis, the dermis, and subcutaneous tissue. The epidermis, which has a thickness of about 0.2 mm, has few blood vessels. The dermis and the subcutaneous tissue have a total thickness of about 2 mm and have many blood vessels. If light reflected or scattered by the surface of the skin such as the epidermis is detected, the light may cause noise in a detection result. According to the reflective optical sensor of the present embodiment, noise due to light from the surface of a target object is not detected because the non-sensing distance d is set to 0.2 mm or greater, and a signal from a target measurement site (blood in a blood vessel) can be efficiently detected because the non-sensing distance d is set to 0.3 mm or less.

In FIG. 3, "n1" represents a refractive index of the transparent resin 25, and "n2" represents a refractive index of a measurement target. "θ1" and "θ2" show angles that define the non-sensing region A1, and represent a maximum incident angle θ1 on the light-emitting side and a maximum exit angle θ2 on the light-emitting side, respectively. "θ3" and "θ4" show angles that define the non-sensing region A2, and represent a maximum incident angle θ4 on the light-receiving side and a maximum acceptance angle θ3 on the light-receiving side, respectively. θ1 and θ3 are determined by the dimensions of the light-emitting element and the light-receiving element, the shapes of the light-emitting region and the light-receiving region, the height of the light-shielding wall, and the distance between each element and the light-shielding wall, and θ2 and θ4 are determined by θ1 and θ3 and n1 and n2. The non-sensing distance d is determined by θ2, θ4, and the thickness t of the light-shielding wall.

The materials of the substrate 11, the light-shielding wall 14, and the light-shielding frame 21 are not particularly limited and can be appropriately selected according to the purpose, but at least one member is preferably made of glass epoxy, and more preferably all members are made of glass epoxy. The use of glass epoxy enables machining by cutting in the manufacture of reflective optical sensors. Since it is not necessary to use a mold for resin molding, it is advantageous in terms of manufacturing processing and cost, especially in the case of small-volume production.

The substrate 11 is preferably a glass epoxy substrate having a light transmittance of 1% or less in the thickness of the substrate 11. The light-shielding wall 14 is preferably a glass epoxy substrate having a light transmittance of 1% or less through the thickness of the light-shielding wall 14. In particular, when the light transmittance through the thickness of the light-shielding wall 14 is 1% or less, transmission of light from the light-emitting element 12 to the light-receiving element 13 through the light-shielding wall 14 can be prevented, and detected noise can be reduced. Herein, the "light transmittance through the thickness of the substrate" means a light transmittance in the thickness direction of a substrate having a specific thickness. The "light transmittance through the thickness of the light-shielding wall" means a light transmittance in the thickness direction of a light-shielding wall having a specific thickness, and the thickness of the light-shielding wall is a direction perpendicular to the thickness direction of the substrate. Similarly, a light-shielding frame having a specific thickness is defined.

A glass epoxy substrate has different optical characteristics in the thickness direction (lamination direction) and the in-plane direction, and a general glass epoxy substrate may have low light-shielding properties in the in-plane direction. An example of the glass epoxy substrate having high light-shielding properties and low light transmittance in the thickness direction and the in-plane direction includes the black printed wiring board material CS-3667B (manufactured by Risho Kogyo Co., Ltd.) having light-shielding properties. The light transmittance may be suitably selected as long as the light transmittance is 1% or less at the wavelengths of light emission and light reception to be measured, but it is preferable that the light transmittance be 1% or less at wavelengths of 650 nm to 1,310 nm, and it is more preferable that the light transmittance be 1% or less at wavelengths of 250 nm to 2,000 nm.

The light-shielding wall 14 and the light-shielding frame 21 are preferably provided via the adhesive layer 24, which is formed by using a light-shielding adhesive on the substrate 11. The light-shielding adhesive is not particularly limited and may be suitably selected according to the purpose, and an example includes the adhesive sheet AD-7006B (manufactured by Risho Kogyo Co., Ltd.), which has an average thickness of 0.05 mm.

As illustrated in FIG. 2, the reflective optical sensor may have one light-emitting element. As another embodiment, as illustrated in FIG. 5, it may have two or more light-emitting elements provided next to each other. Any of the embodiments may be suitably implemented. For example, as in the case of pulse oximeters, there is a growing number of applications that use multiple light-emitting elements for detection depending on a detection target. To achieve miniaturization, configurations are known in which multiple light-emitting elements and a light-receiving element are implemented within one reflective-type optical sensor package.

As illustrated in FIG. 5, when the reflective optical sensor has two or more light-emitting elements 12a and 12b, these light-emitting elements are provided next to each other so as to satisfy the requirements for the relationship of dₑ + dᵣ < t, and for the non-sensing distance d. In other words, the light-emitting elements 12a and 12b are disposed close to the light-shielding wall 14, and the light-emitting elements 12a and 12b are disposed side by side with respect to the light-shielding wall 14, and no other member is disposed therebetween. With this configuration, it is possible to reduce the spatial dependence of the light-radiation and light-receiving characteristics for each light-emitting element, and at the same time, to enhance the uniformity of the spatially dependent characteristics of light radiation and light reception with respect to a measurement target among two or more light-emitting elements.

In the case where a reflective optical sensor has two or more light-emitting elements, such as in the photoreflector disclosed in Patent Document 1, for example, because the two light-emitting elements (12) are provided in different sections and positioned on opposite sides of the photodiode, the radiation conditions of light radiation from one light-emitting element and those from the other light-emitting element are significantly different. This leads to a problem that the spatially dependent characteristics of light radiation and light reception with respect to a measurement target are different between the two light-emitting elements. In other words, there is a problem in that differences in the locations where the two or more light-emitting elements are disposed on the substrate cause differences in their optical characteristics with respect to the light-receiving element, and these differences lead to sensing of different regions (e.g., areas having different blood densities) of the measurement target, thereby deteriorating the uniformity of measurement among the light-emitting elements.

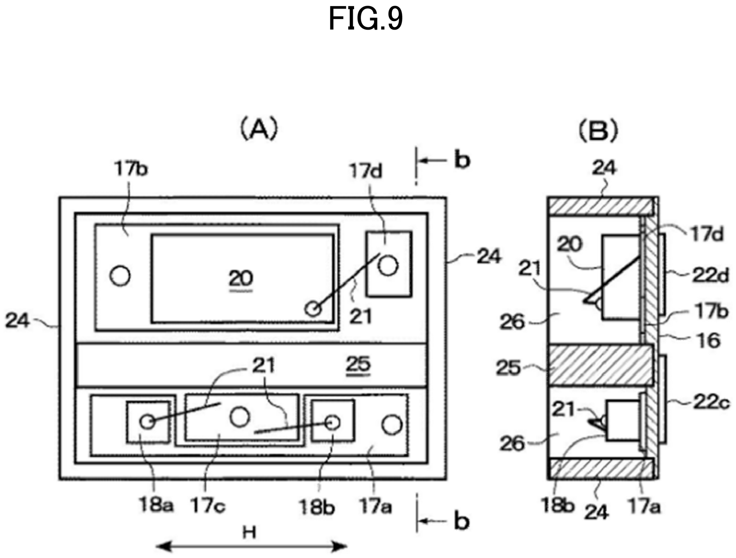

Japanese Unexamined Patent Application Publication No. 2009-038322 discloses a reflective optical sensor having two light-emitting elements in one section. As illustrated in FIG. 9, in the reflective optical sensor disclosed in this prior art document, the light-emitting sections (18a, 18b) and the light-receiving section (20) have light-emitting and light-receiving surfaces disposed in a direction perpendicular to the moving direction (H) of a detection target moving in parallel with the light-emitting and light-receiving surfaces. The light-receiving section (20) receives light reflected by a detection target based on light output from the light-emitting sections (18a, 18b). The light-emitting sections (18a, 18b) are provided with a light-emitting region in which the amount of light emitted at both ends is higher than that at the center of the moving direction (H) of the detection target, so that the detection output of the light-receiving section (20) changes linearly in accordance with the amount of movement of the detection target.

In this reflective optical sensor, although two light-emitting elements are provided in one section, they are provided across a bonding pattern (17c), and the two light-emitting elements are provided at a distance from each other. Therefore, similar to the photoreflector disclosed in Patent Document 1, there is a problem that the directions of light radiation from one of the light-emitting elements and light reception by the light-receiving element and the directions of light radiation from the other light-emitting element and light reception by the light-receiving element are different from each other, resulting in variations of the light-radiation characteristics and light-receiving characteristics with respect to a measurement target across different between spatial positions. On the other hand, according to the reflective optical sensor of the present embodiment having two or more light-emitting elements provided next to each other, the conventional problem can be solved, and the uniformity of the light-radiation and light-receiving characteristics with respect to a measurement target at different spatial positions can be enhanced among the two or more light-emitting elements.

Preferably, the emission wavelengths of the two or more light-emitting elements are different from each other. This makes it possible to detect reflected light having two or more wavelengths, for example, two or more wavelengths that are greatly different wavelengths, and to detect a plurality of pieces of information, detect information based on differences between reflected light having different wavelengths, and correct the information.

The wavelength can be appropriately selected according to information to be measured. Specifically, in a pulse oximeter for measuring blood oxygen saturation, red light (for example, a wavelength of 660 nm) and infrared light (for example, a wavelength of 940 nm) are alternately pulsed, and concentration of oxygenated hemoglobin (HbO₂) can be detected from a difference between reflected light of red light and reflected light of infrared light. In a blood glucose meter, for example, concentration of blood glucose can be measured by correcting reflected light of an absorption wavelength of glucose (peak absorption wavelength of 1,600 nm) by reflected light of an absorption wavelength of water (1,450 nm).

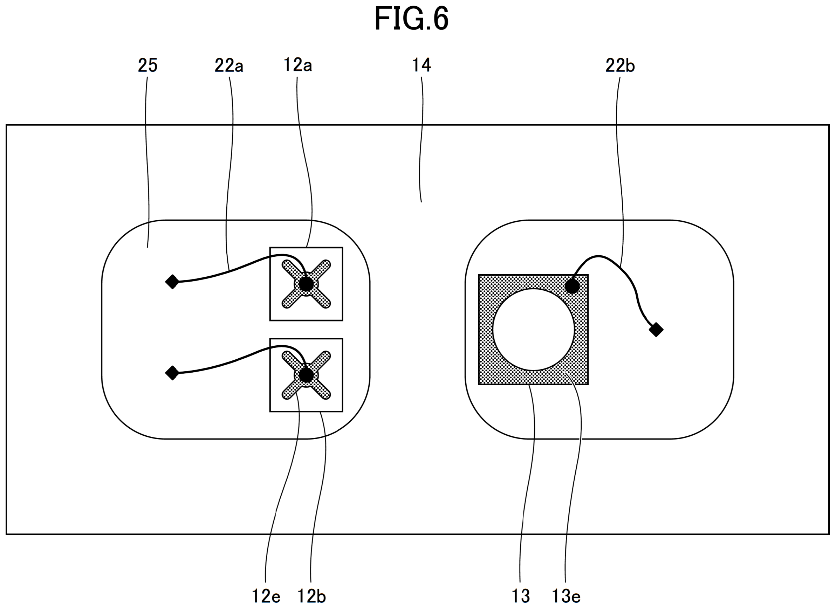

As another embodiment, as illustrated in FIG. 6, the light-shielding frame 21 for separating, together with the light-shielding wall 14, the light-emitting element 12 and the light-receiving element 13 is provided on the substrate 11, and the shape of each section defined by the light-shielding wall 14 and the light-shielding frame 21 is a chamfered square columnar shape. When the shape of each of the sections is a chamfered square columnar shape, the aperture can be reduced. When the aperture is reduced, a stray light component of light emission and light reception is reduced, so that noise can be reduced. When a substrate such as a glass epoxy substrate is used as the light-shielding wall 14 and the light-shielding frame 21, cutting processing becomes easy, which is preferable in terms of processing and cost.

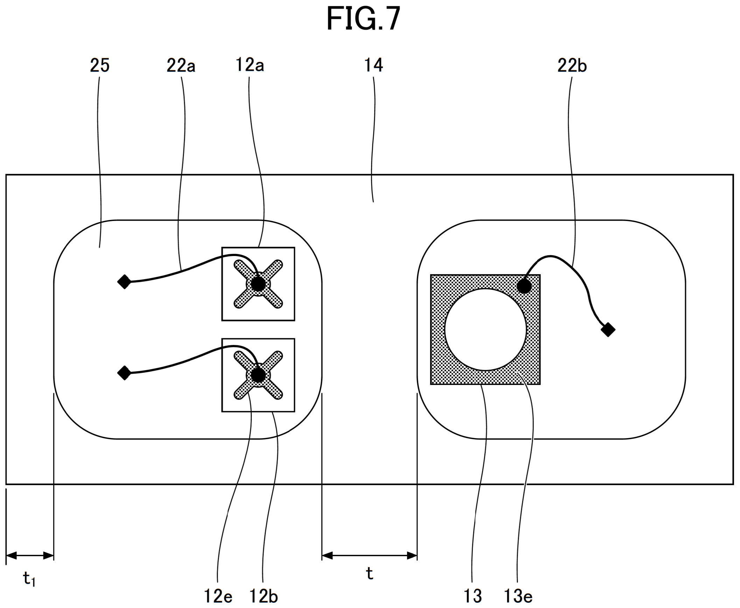

As illustrated in FIG. 7, an embodiment in which the thickness t₁ of the light-shielding frame 21 is smaller than the thickness t of the light-shielding wall 14 can also be suitably implemented. The above embodiment can be easily manufactured by processing such as cutting with a thick dicing blade at the time of element separation. The thickness t₁ of the light-shielding frame 21 can be reduced, and the package size of the reflective optical sensor 10 can be reduced.

### <Examples>

### (Example 1)

As an example of the reflective optical sensor having two light-emitting elements illustrated in FIG. 5, a pulse oximeter for measuring blood oxygen saturation was manufactured. Specifically, a red LED chip (PR7A, manufactured by DOWA Holdings Co., Ltd.) and an infrared LED chip (FP6E, manufactured by DOWA Holdings Co., Ltd.) were used as two light-emitting elements that emit red light (a wavelength of 660 nm) and infrared light (940 nm), and a PD chip used in a large-area photodiode (KPD31S, manufactured by Kyoto Semiconductor Co., Ltd.) having a detection wavelength of 400 nm to 1,100 nm was used as a light-receiving element with a reduced size and light-receiving area. The pulse oximeter of Example 1 was manufactured using a glass epoxy substrate (the black printed wiring board material, CS-3667B, manufactured by Risho Kogyo Co., Ltd.) having a light transmittance of 1% or less as a light-shielding wall and a light-shielding frame, and a light-shielding adhesive sheet (AD-7006B, manufactured by Risho Kogyo Co., Ltd.) as a light-shielding adhesive.

Each of the light-emitting elements was 0.24 mm × 0.24 mm to 0.28 mm × 0.28 mm in size and 0.15 mm in thickness. The light-receiving element was 2.0 mm × 0.7 mm in size and 0.3 mm in thickness, and the size of the light-receiving area was 1.8 mm × 0.5 mm. The light-shielding wall was 0.8 mm in thickness t and 0.8 mm in height. The distance dₑ between the light-shielding wall and the light-emitting elements and the distance dᵣ between the light-shielding wall and the light-receiving element was 0.2 mm, and therefore dₑ + dᵣ < t was satisfied. When the refractive index of the transparent resin was 1.5 and the refractive index of the human body as a measurement target was 1.4, the non-sensing distance d was approximately 0.24 mm.

In the pulse oximeter of Example 1, two light-emitting elements are alternately pulsed, and the concentration of oxygenated hemoglobin (HbO₂) can be detected from the difference between the reflected lights.

### (Example 2)

As an example of the reflective optical sensor having two light-emitting elements illustrated in FIG. 5, a blood glucose meter for measuring blood glucose concentration was manufactured. Specifically, an infrared LED chip used in an infrared LED (KEDE1652H, manufactured by Kyoto Semiconductor Co., Ltd., with a peak emission wavelength of 1,650 nm) was used as one light-emitting element that emits light at the absorption wavelength of glucose (peak absorption wavelength: 1,600 nm), an infrared LED chip used in an infrared LED (KEDE1452H, manufactured by Kyoto Semiconductor Co., Ltd., with a peak emission wavelength of 1,450 nm) was used as another light-emitting element that emits light at the absorption wavelength of water (1,450 nm), and a chip used in a photodiode (InGaAs photodiode, KPDE300, manufactured by Kyoto Semiconductor Co., Ltd.) capable of detecting 80% or greater of a wavelength from 1,200 nm to 1,700 nm was used as a light-receiving element. The blood glucose meter according to Example 2 was manufactured using a glass epoxy substrate (the black printed wiring board material, CS-3667B, manufactured by Risho Kogyo Co., Ltd.) having a light transmittance of 1% or less as a light-shielding wall and a light-shielding adhesive sheet (AD-7006B, manufactured by Risho Kogyo Co., Ltd.) as a light-shielding adhesive.

The light-emitting elements were 0.35 mm × 0.35 mm in size and 0.33 mm in thickness. The light-receiving element was 0.44 mm × 0.44 mm in size and 0.15 mm in thickness, and a diameter of a light-receiving area was 0.3 mm. The light-shielding wall was 0.8 mm in thickness t and 0.8 mm in height. The distance dₑ between the light-shielding wall and the light-emitting elements and the distance dᵣ between the light-shielding wall and the light-receiving element was 0.3 mm, and the relationship dₑ + dᵣ < t was satisfied. When calculated with a refractive index of 1.5 of the transparent resin and a refractive index of 1.4 of the human body to be measured, the non-sensing distance d was approximately 0.27 mm.

In the blood glucose meter of Example 2, the reflected light of the absorption wavelength of glucose (peak absorption wavelength 1,600 nm) is corrected by the reflected light of the absorption wavelength of water (1,450 nm), so that glucose can be detected with high accuracy without being affected by water, and therefore the blood glucose concentration can be measured.

Each of the reflective optical sensors of Examples 1 and 2 does not need to use a mold for resin molding in manufacturing, and has a simple structure. The angle formed by the outgoing angle of light radiation from the light-emitting element and the incident angle of light reception to the light-receiving element can be reduced, and therefore, the spatial dependency of the light-radiation characteristics and the light-receiving characteristics can be decreased. Since the non-sensing distance d is set within a range of 0.2 mm or greater and 0.3 mm or less, noise due to light from the surface of a living body as a measurement target can be reduced, and at the same time, a signal due to light from blood in the living body can be strongly detected.

This international application claims priority from Japanese Patent Application No. 2023-098321 filed on June 15, 2023, and the entire contents thereof are incorporated herein by reference.

### REFERENCE SIGNS LIST

- 10: Reflective optical sensor
- 11: Substrate
- 12: Light-emitting element
- 13: Light-receiving element
- 14: Light-shielding wall
- 21: Light-shielding frame
- 22a, 22b: Conductive wire
- 23a, 23b: Conductive portion
- 24: Adhesive layer
- 25: Transparent resin
- 26: Adhesive layer
- A1: Region
- A2: Region

## Claims

1. A reflective optical sensor comprising:
a substrate;
a light-emitting element and a light-receiving element, both of which are provided on the substrate; and
a light-shielding wall provided between the light-emitting element and the light-receiving element, wherein
a distance (dₑ) between the light-emitting element and the light-shielding wall and a distance (dᵣ) between the light-receiving element and the light-shielding wall, and a thickness (t) of the light-shielding wall satisfies the following relationship dₑ + dᵣ < t,
a shortest distance between a region that does not belong to any of a region in which light emitted from the light-emitting element does not reach a measurement target and a region in which light reflected or scattered by the measurement target does not enter the light-receiving element, and a sensor surface in which the reflective optical sensor and the measurement target are in contact, is 0.2 mm or greater and 0.3 mm or less.

2. The reflective optical sensor according to claim 1, wherein
the light-emitting element is two or more light-emitting elements provided next to each other, and
emission wavelengths of the two or more light-emitting elements are different from each other.

3. The reflective optical sensor according to claim 1 or 2, further comprising:
a light-shielding frame that, together with the light-shielding wall, separates the light-emitting element and the light-receiving element, the light-shielding frame being provided on the substrate, wherein
a shape of each section defined by the light-shielding wall and the light-shielding frame is a chamfered square columnar shape.

4. The reflective optical sensor according to claim 3, wherein
a thickness of the light-shielding frame is smaller than a thickness of the light-shielding wall.

5. The reflective optical sensor according to any one of claims 1 to 5, wherein
the substrate is a glass epoxy substrate having a light transmittance of 1% or less through a thickness of the substrate,
the light-shielding wall is a glass epoxy substrate having a light transmittance of 1% or less with through a thickness of the light-shielding wall, and
the light-shielding wall is provided on the substrate via a light-shielding adhesive.
